# EUROPEAN PATENT SPECIFICATION

(11) **EP 0 634 134 B1**
(45) Date of publication and mention of the grant of the patent: **20.01.1999**
(21) Application number: 94109491.4
(22) Date of filing: 20.06.1994
(51) Int. Cl.: G06F 17/00

(54) **Method and device for enhancing the signal-to-noise ratio of ECG signals**
Methode und Vorrichtung zum Erhöhen des Signal-zu-Rausch Verhältnisses von EKG-Signalen
Méthode et dispositif pour augmenter le rapport signal sur bruit dans des signaux d'ECG

(30) Priority: 16.07.1993 SE 9302436
(43) Date of publication of application: 18.01.1995
(73) Proprietor: Siemens-Elema AB, 171 95 Solna 1 (SE)
(72) Inventor: Ohlsson, Thomas, S-165 66 Hässelby (SE); Atarius, Roozbeh, S-224 68 Lund (SE); Sörnmo, Leif, S-224 65 Lund (SE)

(56) References cited:
- EP-A- 0 424 607
- US-A- 4 802 491
- US-A- 5 226 424
- IEEE TRANSACTIONS ON BIOMEDICAL ENGINEERING, Volume 38, No. 6, June 1991, Raimon Jan et al, "Alignment Methods for Averaging of High-Resolution Cardiac Signals: A Comparative Study of Performance pp 571-579"
- Methods of Information in Medicine, Volume 31, 1992, G. J. H. Uijen et al, "The Performance of Information-Theoretic Criteria in Detecting the Number of Independent Signals in Multilead ECGs"

## Description

The present invention relates to a method and a device for enhancing the signal-to-noise ratio of ECG signals according to the preambles of claims 1 and 8 respectively.

ECG signals recorded on the exterior of the body always contain extensive noise originating from muscular activity, digitalization of the recorded analog ECG-signal, the recording instruments and influence from the power supply. Using modern technology, however, the two latter sources of noise can be largely eliminated. But noise from the muscular activity of the patient and the digitalization procedure often drowns out low-amplitude signal components in the ECG signal.

In an effort to reduce noise and, thus, improve the signal-to-noise ratio for these low-amplitude signal components, the ECG signals have hitherto been subjected to different kinds of averaging procedures. Time-varying Wiener filtration has also been attempted in order to enhance the signal-to-noise ratio, see Weerd and Martens: "Theory and Practice of A Posteriori Wiener Filtering of Average Evoked Potentials", Biol. Cybern., 30, pp. 81-94, (1978), and Weerd and Kap.: "A Posteriori TimeVarying Filtering of Averaged Evoked Potentials", Biol. Cybern., 41, pp. 223-234, (1981). This technic is based on the estimated signal and noise effects determined for different frequencies. In this process, some spectral leakage is unavoidable in the Fourier transform procedure utilized. With low signal-to-noise ratios, this method also results in consistently erroneous estimation of the signal in the noise.

The object of the present invention is to propose a new method and provide a new device which makes possible additional enhancement of the signal-to-noise ratio which, in its turn, makes possible estimation of low-amplitude signal components in the ECG signal, such as so-called "late potentials", without use of Fourier transform with all its disadvantages.

This object is achieved with a method according to claim 1 and a device according to claim 8.

In the present invention representative parts of ECG-signals from a plurality of cardiac cycles are thus filtered in a filter the characteristics of which are related to the prevailing signal-to-noise ratio.

Thus according to the invention, the signals from a plurality of cardiac cycles are time-synchronized to form a signal ensemble, and the correlation across the signal ensemble is determined, although not the time correlation. In this way the size of the resulting cross-covariance matrix is reduced such that determination of the optimum filter at every point of time is made possible.

According to an advantageous embodiment of the device according to the invention, the correlation between the signals in the signal ensemble is determined in successive, overlapping intervals with the aid of a window unit. The optimum filter at any given moment is thus determined through short-period analysis. In this way time distortion is avoided (smearing).

An exemplified embodiment of the device according to the invention will now be described in greater detail, referring to the accompanying drawings on which
FIG. 1 is an overview block diagram of the device according to the invention;
FIG. 2 is a block diagram showing a part of the device in FIG. 1 in greater detail and
FIG. 3 shows curves illustrating the technic utilized by the invention.

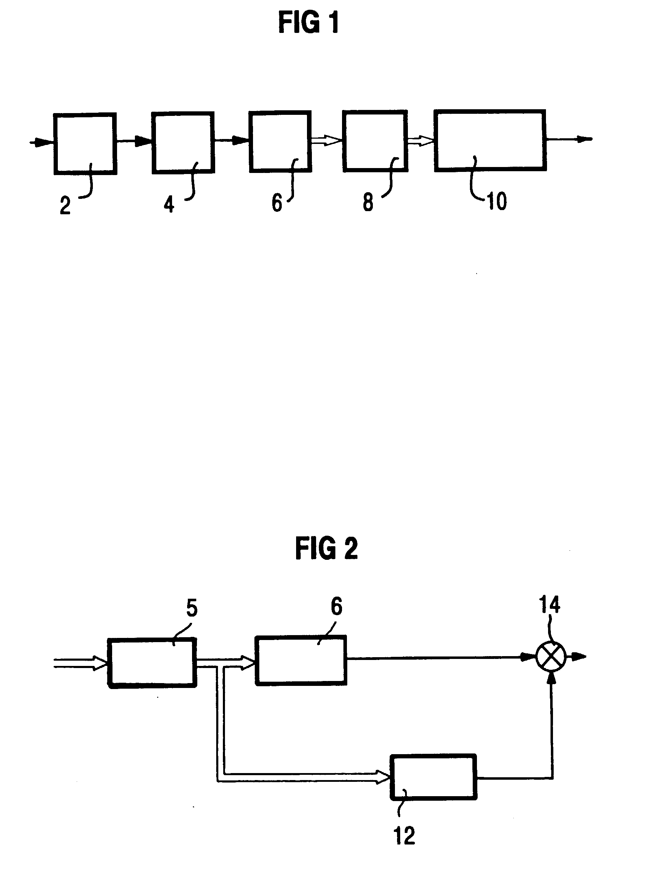

Recorded ECG signals are converted into digital form in the A/D converter 2, and QRS complexes are detected in the detector 4 in FIG. 1. Averaged signal values are formed in the averager 6, and an interval or time window, utilized in the subsequent unit 10 for enhancing the signal-to-noise ratio of ECG signal during at least a part of each cardiac cycle, is determined in the window unit 8.

The units 6, 8 and 10 are described in greater detail with reference to FIG. 2.

Recorded ECG-signals from a plurality of cardiac cycles are time-synchronized so they form an ensemble of signals. The elimination of low-frequency components, which is performed by a high-pass filter with a low cut-off frequency, is essential in the determination of the optimum filter at any given moment. This high-pass filtration, like any other signal processing, takes place in the signal processing unit 5.

In the averager 6 average values over the signal ensemble are formed. Averaging needs not be performed as a "pure" averaging of all the signals in the ensemble, but can be carried out in different other ways in order to "improve" the average value and provide a representative signal cycle. So, e.g. strongly deviating signals in the ensemble could be omitted in the averaging, and the averaging could be performed e.g. in successive, overlapping intervals determined by the window unit 8. Averaging could also consist in median determination.

In the correlation determination unit 12 the correlation between the individual signals in the ensemble of signals is determined according to a Maximum Likelihood method. The ensemble correlation thereby obtained is given by the following equation: 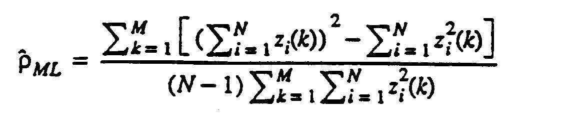 in which zᵢ denotes the i-th filtered ECG signal,
- N: the number of cardiac cycles considered and
- M: the number of samples in the interval or time window.

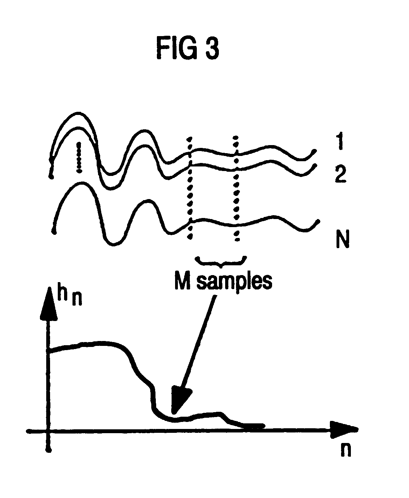

The ensemble correlation thus determined is utilized for determining the optimum filter 14 for filtering the mean signal value obtained from the averager 6 in order to get as large a signal-to-noise ratio as possible. The optimum filter is determined for each successive interval or time window, whereby the resulting filter becomes time-dependent. The filter consequently has a certain weight for each observation interval or sample. The filtration in the unit 14 is thereby reduced to multiplication by a weight function hₙ obtained from the correlation determination unit 12. This is illustrated in FIG. 3 which shows N signals in the signal ensemble and the resulting weight function hₙ which determines the characteristics of the filter 14.

The momentarily optimum filter realized in this way is a nonfrequency-selective filter, and the magnitude of the signalto-noise ratio is used for determining the filter weights. Since the signal-to-noise ratio is a function of the number of available cardiac cycles, also the momentarily optimum filter, and accordingly its output signal, becomes a function of this parameter. This filter can obviously vary considerably if the number of cardiac cycles is low. However, this variation decreases as the number of utilized cardiac cycles increases.

With an increasing number of cardiac cycles N, the noise in the averaged signal decreases, a circumstances which contributes to enhancement of the signal-to-noise ratio at the same time as the momentarily optimum filter thus is improved through the access to more information, as mentioned above. Both these effects consequently contribute to a reduction of fluctuations in the output signal from the filter as the number of cardiac cycles increases.

## Claims

1. Method for enhancing the signal-to-noise ratio of ECG signals, **characterized in** that A/D-converted signals from a plurality of cardiac cycles are time-synchronized to form a signal ensemble, whereupon the concordance between the signals in the ensemble is determined in time intervals, comprising at least two samples, during at least a part of the cardiac cycles, which concordance is employed to control subsequent filtering of the signals.

2. Method according to claim 1, **characterized in** that the concordance is expressed in weights.

3. Method according to claim 1 or 2, **characterized in** that the mean value is formed of the signals in said signal ensemble.

4. Method according to any of the claims 1 - 3, **characterized in** that the determination of concordance comprises determination of the correlation between the signals in the signal ensemble.

5. Method according to claim 4, **characterized in** that the correlation between the signals in said ensemble is determined in successive, overlapping intervals.

6. Method according to claim 3 and 4, **characterized in** that the signal mean value is filtered according to a weight function determined by the correlation.

7. Method of claim 6, **characterized in** that the weight function determines a filter with time variable characteristics for filtering the signal mean value.

8. Device for enhancing the signal-to-noise ratio of ECG-signals, comprising means (2, 4, 5) for recording, A/D-converting and other processing of the ECG- signals, **characterized in** that a synchronization unit is arranged to time-synchronize the signals from a plurality of cardiac cycles to form a signal ensemble, and in that a calculator unit (10, 12) controlled by a window unit (8) is arranged to determine the concordance between the signals in the ensemble in time intervals, comprising at least two samples, during at least a part of the cardiac cycles, which calculator unit (10, 12) is adapted to provide an output dependent on the determined concordance to control subsequent filtering of the signals in a filter unit (14).

9. Device according to claim 8 **characterised in** that the filter unit (14) operates to filter signals according to a weight function determined by the concordance.

10. Device according to claim 8 or 9, **characterized in** that an averager (6) controlled by the synchronization unit or the window unit is arranged to form the mean value of the signals in said ensemble.

11. Device according to any of the claims 8 - 10, **characterized in** that the calculator unit (12) is arranged, for the determination of the concordance, to determine the correlation between the signals in the signal ensemble.

12. Device according to claim 11, **characterized in** that said window unit (8) is arranged to control the calculator unit (12) to determine the ensemble correlation in successive, overlapping intervals of the signals in said ensemble.

13. Device according to any of the claims 10 - 12, **characterized in** that the calculator unit (12) generates a weight function which determines a time-varying characteristic for the filter unit for filtering the signal mean value.

14. Device according to any of the claims 11 - 13, **characterized in** that the calculator unit (12) comprises a Maximum Likelihood estimator.

## Patentansprüche

1. Methode zum Erhöhen des Signal-zu-Rausch Verhältnisses von EKG-Signalen, **dadurch gekennzeichnet,** daß A/D-gewandelte Signale von einer Vielzahl von Herz zyklen zeitsynchronisiert werden, um eine Signalgesamtheit zu bilden, woraufhin die Konkordanz zwischen den Signalen in der Gesamtheit in Zeitintervallen bestimmt wird, die zumindest während mindestens eines Teils der Herz zyklen zwei Proben aufweisen, und daß die Konkordanz dazu verwendet wird, die nachfolgende Filterung der Signale zu steuern.

2. Methode nach Anspruch 1, **dadurch gekennzeichnet**, daß die Konkordanz in Gewichten ausgedrückt wird.

3. Methode nach Anspruch 1 oder 2, **dadurch gekennzeichnet**, daß der Mittelwert der Signale in der Signalgesamtheit gebildet wird.

4. Methode nach einem der Ansprüche 1 - 3, **dadurch gekennzeichnet**, daß die Bestimmung der Konkordanz die Bestimmung der Korrelation zwischen den Signalen in der Signalgesamtheit umfaßt.

5. Methode nach Anspruch 4, **dadurch gekennzeichnet,** daß die Korrelation zwischen den Signalen in der Gesamtheit in aufeinanderfolgenden, sich überlappenden Intervallen bestimmt wird.

6. Methode nach Anspruch 3 und 4, **dadurch gekennzeichnet,** daß der Signalmittelwert gemäß einer durch die Korrelation bestimmte Gewichtsfunktion gefiltert wird.

7. Methode nach Anspruch 6, **dadurch gekennzeichnet**, daß die Gewichtsfunktion ein Filter mit Zeitvariablencharakteristikas zum Filtern des Signalmittelwertes bestimmt.

8. Vorrichtung zum Erhöhen des Signal-zu-Rausch Verhältnisses von EKG-Signalen mit Mitteln (2, 4, 5) zum Aufzeichnen, A/D-Wandeln und zu anderer Bearbeitung der EKG-Signale, **dadurch gekennzeichnet**, daß eine Synchronisationseinheit zum Zeitsynchronisieren der Signale aus einer Vielzahl von Herz zyklen vorgesehen ist, um eine Signalgesamtheit zu bilden, und daß eine durch eine Fenstereinheit (8) gesteuerte Berechnungseinheit (10, 12) vorgesehen ist, um die Konkordanz zwischen den Signalen in der Gesamtheit in den Zeitintervallen zu bestimmen, die zumindest während mindestens eines Teils der Herz zyklen zwei Proben aufweisen, wobei die Berechnungseinheit (10, 12) angepaßt ist, um ein von der bestimmten Konkordanz abhängiges Ausgangssignal zu erzeugen, um die nachfolgende Filterung der Signale in einer Filtereinheit (14) zu steuern.

9. Vorrichtung nach Anspruch 8, **dadurch gekennzeichnet**, daß die Filtereinheit (14) derart arbeitet, daß die Signale gemäß einer durch die Konkordanz bestimmten Gewichtsfunktion gefiltert werden.

10. Vorrichtung nach Anspruch 8 oder 9, **dadurch gekennzeichnet,** daß ein durch die Synchronisationseinheit oder die Fenstereinheit gesteuerter Mittelwertbildner (6) vorgesehen ist, um den Mittelwert der Signale in der Gesamtheit zu bilden.

11. Vorrichtung nach einem der Ansprüche 8 - 10, **dadurch gekennzeichnet,** daß die Berechnungseinheit (12) zur Bestimmung der Konkordanz dazu ausgebildet ist, um die Korrelation zwischen den Signalen in der Signalgesamtheit zu bestimmen.

12. Vorrichtung nach Anspruch 11, **dadurch gekennzeichnet**, daß die Fenstereinheit (8) dazu vorgesehen ist, die Berechnungseinheit (12) zu steuern, um die Korrelation der Gesamtheit in aufeinanderfolgenden, sich überlappenden Intervallen der Signale in der Gesamtheit zu bestimmen.

13. Vorrichtung nach einem der Ansprüche 10 - 12, **dadurch gekennzeichnet**, daß die Berechnungseinheit (12) eine Gewichtsfunktion erzeugt, die ein Zeitvariablencharakteristikum für die Filtereinheit zum Filtern des Signalmittelwertes bestimmt.

14. Vorrichtung nach einem der Ansprüche 11 - 13, **dadurch gekennzeichnet,** daß die Berechnungseinheit (12) einen Kalkulator für die maximale Wahrscheinlichkeit aufweist.

## Revendications

1. Procédé pour améliorer le rapport signal bruit de signaux ECG, caractérisé en ce que des signaux convertis A/N issus d'une pluralité de cycles cardiaques sont synchronisés dans le temps pour former un ensemble de signaux, la concordance entre les signaux de l'ensemble étant alors déterminée en intervalles de temps, comportant au moins deux échantillons, pendant au moins une partie des cycles cardiaques, la concordance étant utilisée pour commander un filtrage ultérieur des signaux.

2. Procédé suivant la revendication 1, caractérisé en ce que la concordance est exprimée en poids.

3. Procédé suivant la revendication 1 ou 2, caractérisé en ce qu'on forme la valeur moyenne des signaux de l'ensemble.

4. Procédé suivant l'une quelconque des revendications 1 à 3, caractérisé en ce que la détermination de la concordance comprend l'étape de détermination de la corrélation entre les signaux de l'ensemble de signaux.

5. Procédé suivant la revendication 4, caractérisé en ce que la corrélation entre les signaux de l'ensemble est déterminée dans des intervalles successifs se chevauchant.

6. Procédé suivant la revendication 3 ou 4, caractérisé en ce que la valeur moyenne de signal est filtrée conformément à une fonction de pondération déterminée par la corrélation.

7. Procédé suivant la revendication 6, caractérisé en ce que la fonction de pondération détermine un filtre ayant des caractéristiques variables dans le temps destiné à filtrer la valeur moyenne de signal.

8. Dispositif pour améliorer le rapport signal bruit de signaux ECG, comportant des moyens (2,4,5) destinés à enregistrer, à convertir A/N les signaux ECG et à effectuer d'autres traitements sur ces signaux ECG, caractérisé en ce qu'une unité de synchronisation est agencée pour synchroniser dans le temps les signaux provenant d'une pluralité de cycles cardiaques pour former un ensemble de signaux, et en ce qu'une unité (10,12) de calculateur commandée par une unité (8) formant fenêtre est agencée pour déterminer la concordance entre les signaux de l'ensemble dans des intervalles de temps, comprenant au moins deux échantillons, pendant au moins une partie des cycles cardiaques, l'unité (10,12) de calculateur étant adaptée pour fournir une sortie fonction de la concordance déterminée pour commander un filtrage ultérieur des signaux dans une unité (14) formant filtre.

9. Dispositif suivant la revendication 8, caractérisé en ce que l'unité (14) formant filtre fonctionne pour filtrer des signaux conformément à une fonction de pondération déterminée par la concordance.

10. Dispositif suivant la revendication 8 ou 9, caractérisé en ce qu'un dispositif (6) de formation de moyenne commandé par l'unité de synchronisation ou l'unité formant fenêtre est agencée pour former la valeur moyenne des signaux de l'ensemble.

11. Dispositif suivant l'une quelconque des revendications 8 à 10, caractérisé en ce que l'unité (12) formant calculateur est agencée, pour la détermination de la concordance, pour déterminer la corrélation entre les signaux de l'ensemble.

12. Dispositif suivant la revendication 11, caractérisé en ce que l'unité (8) formant fenêtre est agencée pour commander l'unité (12) formant calculateur pour déterminer la corrélation d'ensemble dans des intervalles successifs se chevauchant des signaux de l'ensemble.

13. Dispositif suivant l'une quelconque des revendications 10 à 12, caractérisé en ce que l'unité (12) formant calculateur produit une fonction de pondération qui détermine une caractéristique variant dans le temps pour l'unité formant filtre destinée à filtrer la valeur moyenne de signal.

14. Dispositif suivant l'une quelconque des revendications 11 à 13 caractérisé en ce qu'une unité (12) formant calculateur comprend un estimateur de probabilité maximale.
